(19) Europäisches Patentamt
European Patent Office
Office européen des brevets

(11) Publication number. **0 133 170**
**A2**

(12) **EUROPEAN PATENT APPLICATION**

(21) Application number: **84850211.8**

(22) Date of filing: **04.07.84**

(51) Int. Cl.[4]: **A 61 K 31/70**
A 61 L 2/18, C 12 Q 1/04

(30) Priority: **15.07.83 SE 8304006**

(43) Date of publication of application:
**13.02.85 Bulletin 85/7**

(84) Designated Contracting States:
**AT BE CH DE FR GB IT LI NL SE**

(71) Applicant: **Karlsson, Karl-Anders**
**Nilssons Berg 37**
**S-411 43 Göteborg(SE)**

(71) Applicant: **Lindberg, Alf Anton**
**Johan Baners väg 45**
**S-182 75 Stocksund(SE)**

(72) Inventor: **Karlsson, Karl-Anders**
**Nilssons Berg 37**
**S-411 43 Göteborg(SE)**

(72) Inventor: **Lindberg, Alf Anton**
**Johan Baners väg 45**
**S-182 75 Stocksund(SE)**

(74) Representative: **Burman, Tore et al,**
**Bergling & Sundbergh AB P.O. Box 7645**
**S-103 94 Stockholm(SE)**

(54) **A compound and a composition for therapeutic or diagnostic use and a method for therapeutic treatment.**

(57) A composition for therapeutic or diagnostic use in connection with conditions due to bacteria, said composition consisting as an active constituent a compound having the formula:

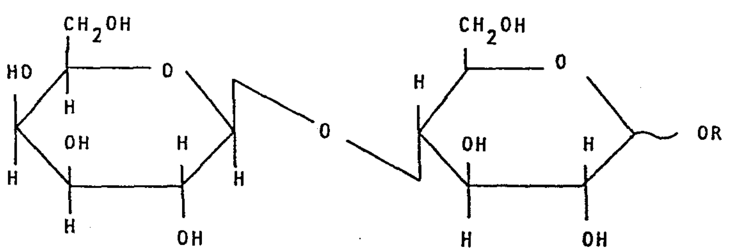

(I)

wherein R is hydrogen or an organic residue;

a method for therapeutic treatment;

a process for the purification of acceptor structures of bacteria; and

a process for carrying out desinfection.

TITLE OF INVENTION

A compound and a composition for therapeutic or diagnostic use and a method for therapeutic treatment.

The present invention relates to compounds and compositions useful for therapeutic treatment of disorders caused by bacteria as well as profylaxis and diagnosis in connection herewith. The invention also covers a method for therapeutic treatment of mammals including man.

The technique according to this invention is applicable to patogenic as well as apatogenic microorganisms of different kinds, particularly bacteria.

There has been calculated (see Savage, D.C, (1977) Annu.Rev.Microbiol. 31, 107-133) that only about 10% of the cells of the human organism are made up of animal cells, whereas the remaining part is constituted by microbial cells mainly residing in the gastrointestinal tract. For a long time adhesion to the host epitheleal cellsurface has been considered essential for the colonisation of normal bacteria as well as infection with patogenic bacteria (see Beachey, E.H. (ed) Bacterial Adherence, Receptors and Recognition, Series B, Vol. 6, Chapman and Hall, London, 1980). It is clear also from the latter reference that one has thought that the bacterial adherence is caused by interaction between a protein-carbohydrate based ligand and receptor, although no such receptor has been structurally identified. Through the present invention there is provided for the first time the structure of the receptor showing activity towards the majority of the bacteria normally found in the human organism.

In accordance with the present invention it has been surprisingly found that the active receptor substance visavis bacteria normally occurring in mammals has the formula:

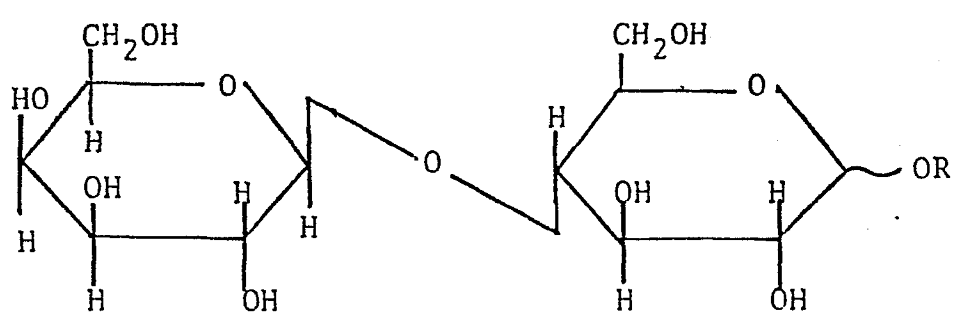

(I)

wherein R is hydrogen or an organic residue. The active entity of the receptor substance is thus constituted by a lactose residue, the reducing end of which may be varied in accordance with the formula given above. It is, however, preferred that OR in the compound of the formula (I) is in β-configuration.

Examples of active substances within formula I are lactose and lactosyl ceramide. R in formula (I) may further be lower alkyl, aryl or a carbohydrate residue, wherein R can be the residue of a natural or synthetic glycoconjugate.

The expression "lower" used in this disclosure relates to a group containing 1-6 carbon atoms, particularly 1-4 carbon atoms and especially 1 or 2 carbon atoms.

Moreover, $R_1$ in formula I may be a macromolecular carrier, optionally including a coupling arm. Such carrier can be a synthetically or naturally occurring polypeptide, polysaccharide or other type of polymer or particle.

The present invention thus has for a primary object to provide a composition or a compound as defined above which can be used diagnostically or can constitute the foundation for a method for therapeutic or profylactic treatment of mammals including man.

Another object of the invention is to provide a process for isolation or purification of bacteria or receptor structures of bacteria.

Furthermore, the invention provides a method for therapeutic treatment of mammals including man, an active amount of a composition or a compound of the type defined above being administered to the mammal.

The present invention can also be used for carrying out desinfections of surfaces, a composition or a compound of the above type being applied to the surface and the composition being then removed with the bacteria adhering thereto.

In regard to the meaning of substituent $R_1$ of the formula I above this may be of any type as long as it does not negatively affect the conditions in connection with the use of the invention. Thus, $R_1$ may be hydrogen, lower alkyl or

lower acyl but $R_1$ may also constitute the residue of a natural or synthetic glycoconjugate, the compound according to the invention thus being a glycoconjugate, for example a glycolipid.

The active substances according to the present invention can be formulated for use in human or veterinary medicine for therapeutic, profylactic or diagnostic uses. In clinical practice the active constituents are normally administered orally or rectally or by injection in the form of a pharmaceutical preparation containing the active constituents in combination with a pharmaceutically acceptable carrier, which may be solid, semisolid or liquid, or as a capsule, and such compositions constitute a further aspect of the invention. The compounds may also be used as such without carrier and in a form of an aqueous solution for injection. As examples of pharmaceutical preparations there may be mentioned tablets, drops, solutions and suppositories. The active substance usually constitutes from 0.05 to 99% by weight of the preparation, for example from 0.1 to 50% for preparations intended for oral administration.

To manufacture pharmaceutical preparations in the form of dose units for oral application containing a compound according to the invention the active constituents can be admixed with a solid, pulverulent or other carrier, for example lactose, saccharose, sorbitol, mannitol, starch, such as potatoe starch, corn starch, amylopectin, a cellulose derivative or gelatin and may also include lubricants, such as magnesium or calcium stearate, or polyethylene glycol waxes compressed to form tablets or cause for dragées.

By using several layers of the active drug separated by slowly dissolving layers tablets of delayed release are obtained.

Liquid preparations for oral application can be in the form of elixires, syrups or suspensions, for example solutions containing from 0.1 to 20% by weight of active substance, sugar and a mixture of ethanol, water, glycerol, propylene, glycol and optionally other additives of a conventional character.

The dose by which the active constituents are administered may vary within wide limits and depend on different factors, such as the severity of the disorder, the age and the weight of the patient and can be individually adjusted. As a conceivable range for the quantity of active constituents that may be administered per day there may be mentioned from 0.1 to 2000 mg or from 1 mg to 2000 mg.

The invention will in the following be further described by non-limiting examples and in connection to the appended drawing, wherein

Fig. 1 shows a thin layer chromatogram indicating the binding of radioactively labelled bacteria, and

Fig. 2 shows a binding curve for a certain bacterium.

In connection with the birth of the invention a new analysis method has been devised which can be defined as a form of chromatographic binding assay. The principle for same is the following.

A thin layer chromatogram is prepared from pure defined receptor strutures and/or receptor structures in partly unknown mixture extracted from tissue of interest. The thin layer chromatogram is prepared on a surface of silica gel and is treated after the application for avoiding unspecific binding of the bacteria of interest with a silica gel. The bacteria which has been radioactively labelled with $J^{125}$ is then transferred to the treated chromatogram. After washing away excess bacteria there is shown by autoradiography how it has been bound to the individual separated glycolipids . In this manner there is obtained information concerning which glycolipids that are active receptors in relation to the applied bacteria and which glycolipids or substances not showing binding capacity.

Far going description of the binding specificity may hereby be obtained by comparision of closely related structures. The identity for a receptor (binding substance) is suitably obtained by comparision with structurally known reference substances. The receptor structures utilized in this disclosure are known substances.

EXAMPLE 1

The experiment was carried out with a thin layer chromatogram on a surface of silica gel prepared as indicated above. The thin layer chromatogram was detected with anisaldehyde, and autoradiogram was performed after binding with iodo-125-labelled bacteria, namely Propionibacterium granulosum and Propionibacterium freudenreichii.

Fig. 1 shows the thin layer chromatograms, the left chromatogram relating to detection with anisaldehyde, the middle chromatogram relating to experiments with Propionibacterium granulosum and the right chromatogram relating to Propionibacterium freudenreichii.

The receptor samples were total non-acidglycolipids of human erytrocytes (1 in the chromatograms)human meconium (2 and 3 in the chromatograms),monkey intestine (4 in the chromatograms), dog small intestine (5 in the chromatograms), rabbit small intestine (6 in the chromatograms), guinea-pig small intestine (7 in the chromatograms) and rat small intestine (8 in the chromatograms). Locations marked with X in the chromatograms are for lactosylceramide with sfingocine and long-chain, non-hydroxi fatty acids (1 and 5 in the chromatograms), lactotetraosylceramide (3 in the chromatograms) and isoglobotriaosylceramide (7 in the chromatograms). The binding pattern as shown for Propionibacterium granulosum (middle chromatogram) was identical for several species of the genus Clostridia, for example C. difficile. Also the pattern for Bacteroides was about the same but binding was relatively weaker to the tri- and tetrameres.

EXAMPLE 2

The bindingcurve for Bacteroides fragilis in microwells visavis pure lactosylceramide, isoglobotriaosylceramide and pentaglycosylceramide of bloodgroup H was determined. The results are given in fig. 2, where the vertical axis relates to radioactivity in impulses per minute, whereas the horisontal axis indicates the content of glycoconjugate in nanograms per well.

As is clear from the figure lactosylceramide results in

substantially higher binding strength than the other glycoconjugates.

EXAMPLE 3

In general mapping of the binding properties of bacteria while using the procedure described in the present disclosure the following bacteria were found to use the active constituent according to the present invention as a receptor for adherence:

Bacteroides 8482
" 10581
" ovatus An 719
" " An 526
" " An 1030
" thetaiotamicron 309
" uniformis 42-4
" fragilis ATCC 23745
" " NCTC 9343
" vulgatus An 1322
" " An 879
" distasonis An 696
" " ATCC 8503

Clostridium difficile 790/81
" " KOHN
" " 295:2
" " F 433:1
" " ATCC 7689
" perfringens typ A ATCC 13124
" botulinum typ A ATCC 25763
" " typ F ATCC 25764

Lactobacillus fermentum ATCC 9338
" acidofilus ATCC 4356

Fusobacterium varium ATCC 8501
" necrophorus ATCC 10953

Propionibacterium acnes
" granulosum
" freudenreichii

Shigella dysenteriae 1 Y 980/82
" " " Y 211/83
" " " Y 281/83
" " " Y 307/83
" " " 3818-0

CLAIMS

1. A composition for therapeutic or diagnostic use in connection with conditions due to bacteria, characterized by containing as an active constituent a compound having the formula:

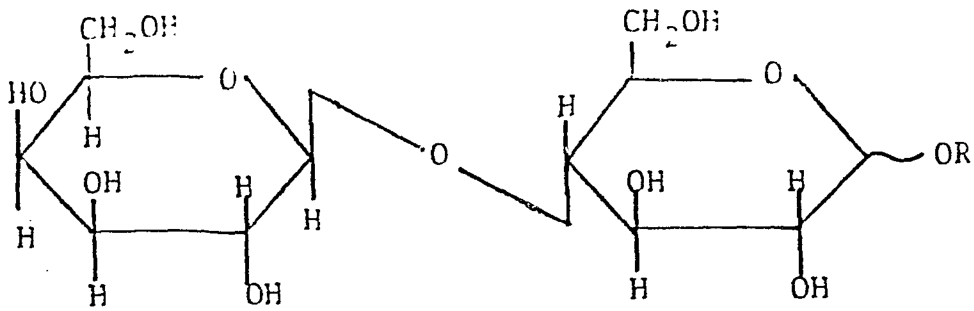

(I)

wherein R is hydrogen or an organic residue.

2. A composition according to claim 1, characterized thereby that the compound is lactose.

3. A composition according to claim 1, characterized thereby that the compound is lactosylceramide.

4. A composition according to claim 1, characterized thereby that R is lower alkyl.

5. A composition according to any preceding claim, characterized thereby that OR is in β-configuration.

6. A composition according to any preceding claim, characterized thereby that the compound (I) is present in combination with a pharmaceutically acceptable carrier.

7. A method for therapeutic treatment of mammals including man, characterized by administering to the mammal an active amount of a composition according to any preceding claim.

8. A process for the purification of acceptor structures of bacteria, characterized by using for the purification the affinity between the compound (I) according to any of claims 1-6 and the acceptor structures in question of the bacteria.

9. A process for carrying out desinfection of surfaces, characterized by applying to the surface a composition according to any of claims 1-6 and then removing the composition with the bacteria adhering thereto.

10. A method for determining the presence of certain bacteria of the gastrointestinal tract of mammals including man, comprising determining the degree of interaction between the bacteria and the compound of any of claims 1-5.

Fig. 1

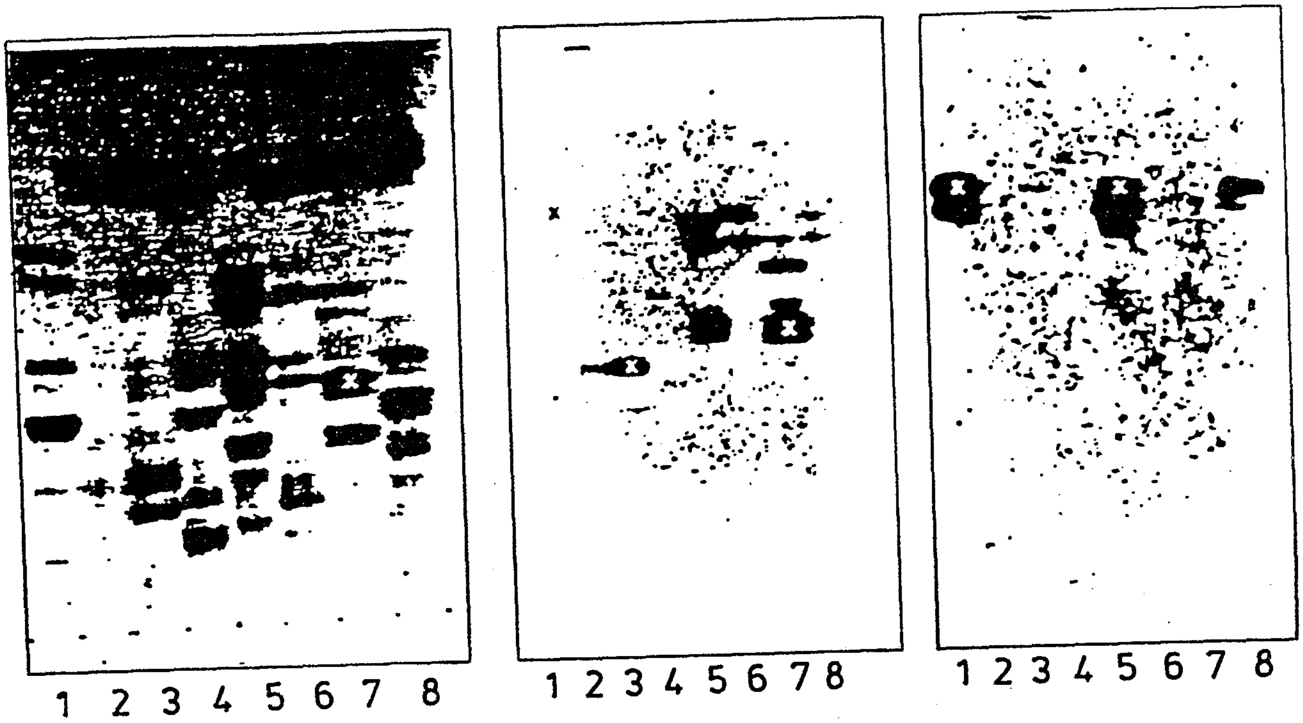
1 2 3 4 5 6 7 8
1 2 3 4 5 6 7 8
1 2 3 4 5 6 7 8

Fig. 2

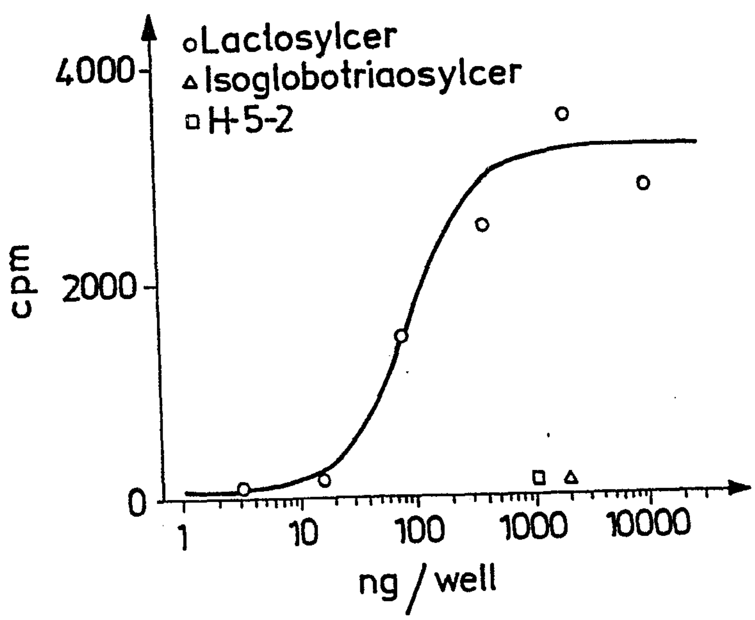
o Lactosylcer
Δ Isoglobotriaosylcer
□ H-5-2
4000
2000
0
cpm
1
10
100
1000
10000
ng / well